# EUROPEAN PATENT SPECIFICATION

(11) **EP 1 476 192 B1**
(45) Date of publication and mention of the grant of the patent: **25.04.2007**
(21) Application number: 03702642.4
(22) Date of filing: 14.02.2003
(51) Int. Cl.: A61K 45/06, A61K 31/519, A61K 31/497, A61P 35/00

(54) **METHODS AND COMPOSITIONS FOR TREATING HYPERPROLIFERATIVE CONDITIONS**
VERFAHREN UND ZUSAMMENSETZUNGEN ZUR BEHANDLUNG VON HYPERPROLIFERATIVEN ZUSTÄNDEN
PROCEDES ET COMPOSITIONS POUR TRAITER DES ETATS HYPERPROLIFERATIFS

(30) Priority: 14.02.2002 US 356912 P
(43) Date of publication of application: 17.11.2004
(73) Proprietor: DANA-FARBER CANCER INSTITUTE, INC., Boston, MA 02115 (US)
(72) Inventor: STILES, Charles, Dean, Newton Center, MA 02459 (US); GARCIA-ECHEVERRIA, Carlos, CH-4052 Basel (CH)
(74) Representative: de Weerd, Petrus G.W.
(86) International application number: PCT/EP2003/001507
(87) International publication number: WO 2003/068265

(56) References cited:
- B.M.F. MOW E.A.: "Effects of the Bcr/abl kinase inhibitors STI571 and adaphostin (NSC 68410) on chronic myelogenous leukemia cells in vitro" BLOOD, vol. 99, no. 2, 2002, pages 664-671, XP001151675
- X.SUN E.A.: "Comparison of effects of the tyrosine kinase inhibitors AG957, AG490, and STI571 on BCR-ABL-expressing cells, demonstrating synergy between AG490 and STI571" BLOOD, vol. 97, no. 7, 2001, pages 2008-2015, XP001151676

## Description

### Technical Field and Background Art

The present invention describes the use of combinations of signal transduction inhibitors that interact in a synergistic way so that the antiproliferative response to the combination exceeds the response to the inhibitors applied individually, for the treatment of glioblastoma.

The proliferation of human and animal cells is regulated by small peptide hormones known as growth factors, chemokines, cytokines or interleukins. Hereafter, these proteins will be referred to collectively as "growth factors". It has long been recognized that the proliferation of human and animal cells *in vitro* is promoted by combinations of growth factors. Typically the combination includes one growth factor that is relatively tissue-specific in action and another growth factor that acts upon a wide range of tissues.

Specific examples of tissue-specific growth factors include platelet-derived growth factor (PDGF), nerve growth factor (NGF), colony stimulating factor 1 (CSF1) and erythropoietin (EPO). These growth factors are tissue selective in action because the corresponding receptor proteins that are required for their function are expressed only in a narrow subset of human and animal tissues. For example, PDGF receptors are expressed in fibroblast cells, smooth muscle and a few other connective tissue elements but not in epithelial cells. NGF receptors are expressed in cells of neural crest origin but not in fibroblasts, smooth muscle or connective tissue.

A specific example of a growth factor that acts upon a wide range of tissues is insulin-like growth factor 1 (IGF1) [IGF1 is also known as non-suppressible insulin-like activity 1 (NSILA1) and also as "somatomedin c". The term IGF1 is currently preferred and is used here. However, in some of the early literature cited here, the term "somatomedin c" is used.]. The specific receptor protein for IGF1 is expressed on virtually all human and animal cells and virtually all cells require activation of IGF receptors to proliferate *in vitro*. For this reason, IGF1 or insulin (which at high concentrations can substitute for IGF1 to activate the IGF1 receptor) is a central component of commercially available culture media that can be used to support cell growth in the absence of serum.

Under pathological conditions, both tissue-specific growth factors and growth factors such as IGF1 have been linked to hyperproliferative disease states. For example, PDGF is linked to certain cancers and to the proliferation of smooth muscle cells within atherosclerotic plaques of the coronary arteries. IGF1 overexpression is linked to acromegaly and to cancer of the prostate.

As a consequence of the way that growth factors function normally to modulate cell proliferation, compounds that inhibit the activation of growth factor receptors on the outer cell surface or inhibit the function of signal generating proteins within the cell can be used to inhibit cell growth.

The inhibition of PDGF receptors has been successfully achieved through the use of a compound identified as STI-571 having the chemical name 4-(4-methylpiperazin-1-ylmethyl)-N-[4-methyl-3-(4-pyridin-3-yl)-pyrimidin-2-ylamino)-phenyl]-benzamide. STI-571 is described in EP 0 564 409 and, in the form of the methane sulfonate salt, especially in the preferred β-crystal form, in WO 99/03854, and is also known as Imatinib, Glivec or Gleevec. Gleevec also inhibits the activation of a lineage-specific growth factor receptor that is closely related to the PDGF receptor - namely the kit receptor. Gleevec further also inhibits the activity of a cytoplasmic signal generating protein known as abl. The abl protein is intimately involved in the pathology of chronic myeloid leukemia in humans. Like the PDGF and kit receptors, abl is a protein with tyrosine-specific protein kinase activity. Gleevec is a new therapeutic for at least temporarily treating cancer. Gleevec, which was approved by the FDA in May 2001, has been shown to be effective in treating chronic myeloid leukemia and gastrointestinal stromal tumours in humans. Gleevec (hereinafter referred to as "STI-571") constitutes an especially useful tissue specific growth factor receptor inhibitor in the context of the present invention.

WO 02/092599 describes compounds which are potent IGF1 receptor inhibitors, among them trans-5-(3-benzyloxy-phenyl)-7-(3-pyrrolidin-1-ylmethyl-cyclobutyl)-7H-pyrrolo[2,3-d]pyrimidin-4-ylamine (hereinafter referred to as "ADW"). These IGF1 receptor inhibitors are particularly useful tissue non-specific growth factor receptor inhibitors in the context of the present invention.

### Summary of the Invention

The present invention relates to a combination which comprises (a) an inhibitor of a tissue non-specific growth factor receptor and (b) an inhibitor of a tissue specific growth factor receptor for simultaneous, concurrent, separate or sequential use, especially for use in the treatment of hyperproliferative conditions, such as in particular cancer, in a mammal, particularly a human. The invention also relates to pharmaceutical compositions comprising such a combination and to a method of treating hyperproliferative conditions, such as especially cancer, with such a combination. The present invention further also relates to a commercial package or product comprising such a combination.

### Brief Description of the Drawings

The foregoing features of the invention will be more readily understood by reference to the following detailed description, taken with reference to the accompanying drawings, in which:

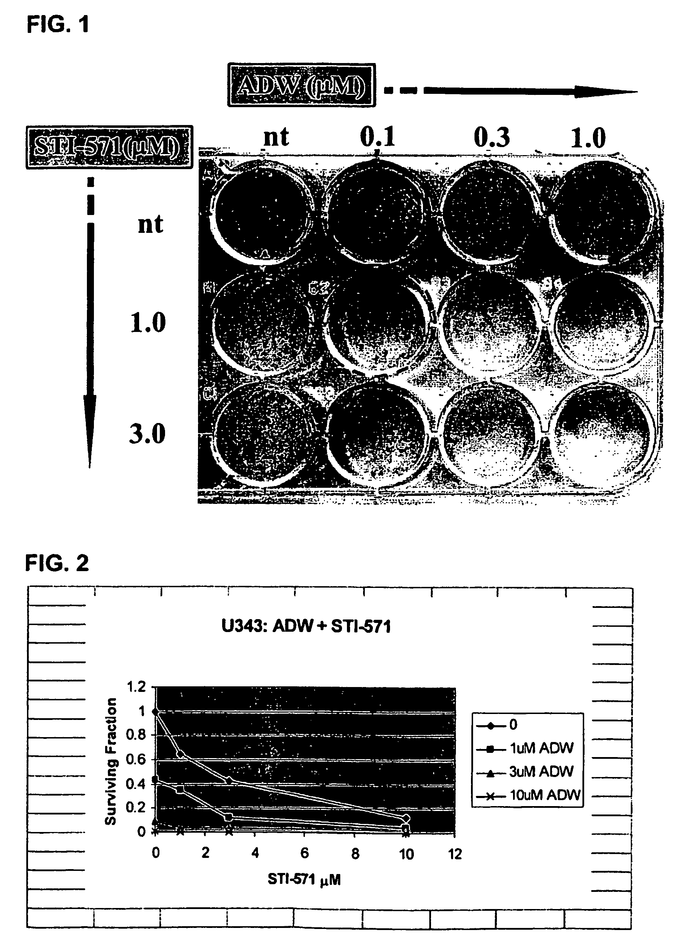

Fig. 1 is a photograph of a 12 well plate containing simian sarcoma virus-transformed NIH/3T3 (sis 3T3; for the preparation thereof see Shamah et al., Mol. Cell. Biol. 13, 7203-7212, 1993; the NIH/3T3 cells are available at ATCC under the ATCC Number CRL-1658) fibroblast cells exposed to varying amounts of ADW and STI-571 and the synergistic effect of these compounds in effecting growth inhibition.
Fig. 2 is a graph showing fractional cell survival determined by cell counting 5 days following combined treatment of the U343 human glioma cell line (Fig. 2) (for U343 cell line see Senger et al, Cancer Research 62, 2131-2140, 2002 and Shamah et al., Mol. Cell. Biol. 13, 7203-7212, 1993). The cell line displays increased killing with combined treatment.

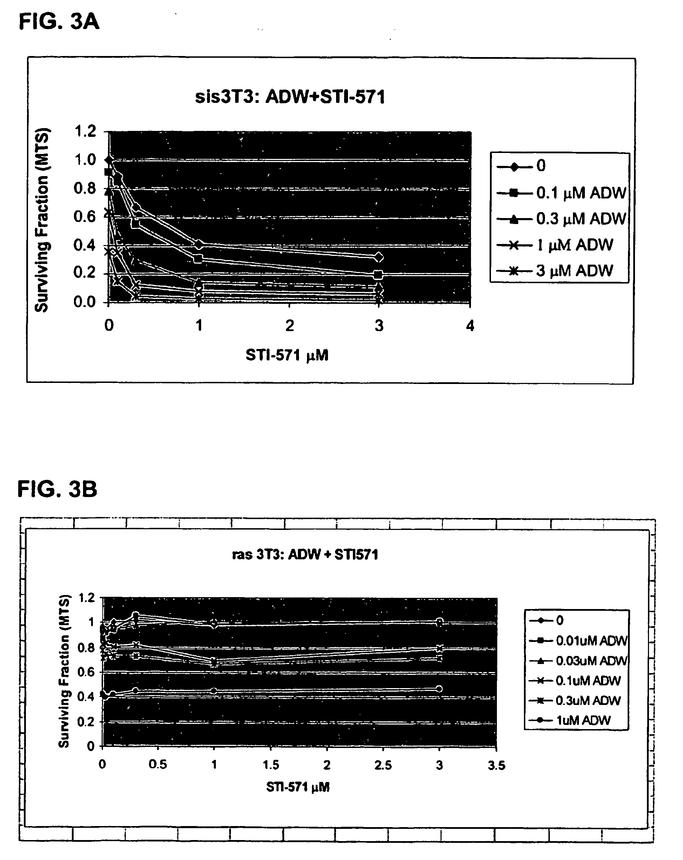

Fig. 3 shows fractional cell survival following combined treatment with ADW/STI-571. Cell survival was determined by the use of an MTS-based assay for cell viability. Combined treatment in sis 3T3 fibroblasts results in increased cell-killing (Fig. 3A). Control ras-transformed fibroblasts (ras 3T3 cells; for the preparation thereof see Feig L.A. and Cooper G.M., Mol. Cell. Biol. 8, 2472-2478, 1988) show decreased survival following treatment with ADW but show no effect on survival with STI-571 administered alone or in combination with ADW over the dose range examined (Fig. 3B).

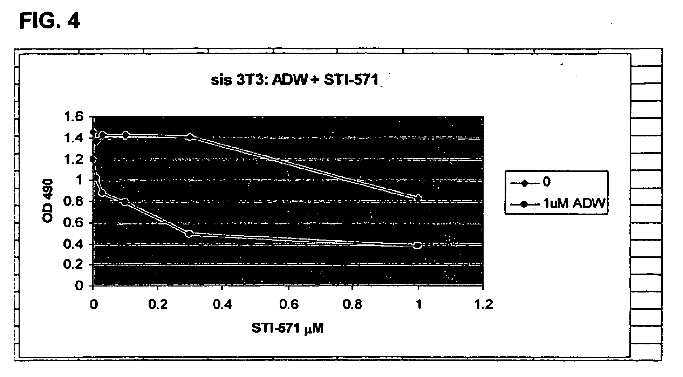

Fig. 4 shows synergism between STI-571 and ADW for growth inhibition of sis 3T3 cells: (quantitative data plus expanded scale for STI-571).

### Detailed Description of Specific Embodiments

Definitions: As used in this description and the accompanying claims, the following terms shall have the meanings indicated, unless the context otherwise requires:

The terms "inhibitor of a tissue non-specific growth factor receptor" and "inhibitor of a tissue specific growth factor receptor" refer to any molecule that reduces the activity of a tissue specific or non-specific growth factor receptor, respectively. Such inhibitors may include for example antagonists that bind to the growth factor receptor or molecules that bind to the growth factor itself to prevent its binding to the cell surface receptor and thus act as growth factor receptor inhibitors. It further also includes inhibitors of the tyrosine kinase activity of growth factor receptors as well as compounds which for example decrease the expression level of growth factors or their receptors or inhibit "downstream" signalling of growth factor receptor activation. The inhibitors may be small synthetic molecules, antisense nucleic acids or macromolecules that may be polypeptides, lipids and/or polysaccharides.

"Tissue non-specific growth factor receptor" refers to, but is not limited to, the IGF-1 receptor, epidermal growth factor receptor and the transforming growth factor receptor.

"Tissue specific growth factor receptor" refers to, but is not limited to, the NGF receptor, fibroblast growth factor receptor, vascular endothelial growth factor receptor, brain derived growth factor receptor and the PDGF receptor.

The present invention relates to combinations comprising (a) an inhibitor of a tissue non-specific growth factor receptor, such as in particular an IGF1 receptor inhibitor, and (b) an inhibitor of a tissue specific growth factor receptor, such as in particular a PDGF receptor inhibitor.

IGF-1 receptor inhibitors include for example: specific antibodies, antisense nucleic acid known in the prior art (for example, inhibitors including tyrphostin described by Blum et al., Biochemistry 2000, Vol. 39, pp. 15705-15712 incorporated herein by reference).

Preferred IGF1 receptor inhibitors to be used in accordance with the present invention are those described in WO 02/092599 and include in particular the following compounds or salts thereof:
cis-7-(3-aminomethyl-cyclobutyl)-5-(3-benzyloxy-phenyl)-7H-pyrrolo[2,3-d]pyrimidin-4-ylamine;
trans-7-(3-aminomethyl-cyclobutyl)-5-(3-benzyloxy-phenyl)-7H-pyrrolo[2,3-d]pyrimidin-4-ylamine;
cis-3-[4-amino-5-(3-benzyloxy-phenyl)-pyrrolo[2,3-d]pyrimidin-7-yl]-cyclobutanecarboxylic acid dimethylamide;
trans-3-[4-amino-5-(3-benzyloxy-phenyl)-pyrrolo[2,3-d]pyrimidin-7-yl]-cyclobutanecarboxylic acid dimethylamide;
cis-3-[4-amino-5-(3-benzyloxy-phenyl)-pyrrolo[2,3-d]pyrimidin-7-yl]-cyclobutanecarboxylic acid methylamide;
trans-3-[4-amino-5-(3-benzyloxy-phenyl)-pyrrolo[2,3-d]pyrimidin-7-yl]-cyclobutanecarboxylic acid methylamide;
cis-5-(3-benzyloxy-phenyl)-7-(3-dimethylaminomethyl-cyclobutyl)-7H-pyrrolo[2,3-d]pyrimidin-4-ylamine;
trans-5-(3-benzyloxy-phenyl)-7-(3-dimethylaminomethyl-cyclobutyl)-7H-pyrrolo[2,3-d]pyrimidin-4-ylamine;
cis-5-(3-benzyloxy-phenyl)-7-(3-methylaminomethyl-cyclobutyl)-7H-pyrrolo[2,3-d]pyrimidin-4-ylamine;
trans-5-(3-benzyloxy-phenyl)-7-(3-methylaminomethyl-cyclobutyl)-7H-pyrrolo[2,3-d]pyrimidin-4-ylamine;
trans-N-{3-[4-amino-5-(3-benzyloxy-phenyl)-pyrrolo[2,3-d]pyrimidin-7-yl]-cyclobutylmethyl}-guanidine;
cis-N-{3-[4-amino-5-(3-benzyloxy-phenyl)-pyrrolo[2,3-d]pyrimidin-7-yl]-cyclobutylmethyl}-guanidine;
trans-N-{3-[4-amino-5-(3-benzyloxy-phenyl)-pyrrolo[2,3-d]pyrimidin-7-yl]-cyclobutylmethyl}-methanesulfonamide;
cis-N-{3-[4-amino-5-(3-benzyloxy-phenyl)-pyrrolo[2,3-d]pyrimidin-7-yl]-cyclobutylmethyl}-methanesulfonamide;
trans-N-{3-[4-amino-5-(3-benzyloxy-phenyl)-pyrrolo[2,3-d]pyrimidin-7-yl]-cyclobutylmethyl}-4-methoxy-benzenesulfonamide;
trans-N-{3-[4-amino-5-(3-benzyloxy-phenyl)-pyrrolo[2,3-d]pyrimidin-7-yl]-cyclobutylmethyl}-4-methyl-benzenesulfonamide;
trans-N-{3-[4-amino-5-(3-benzyloxy-phenyl)-pyrrolo[2,3-d]pyrimidin-7-yl]-cyclobutylmethyl}-4-nitro-benzenesulfonamide;
propane-2-sulfonic acid trans-{3-[4-amino-5-(3-benzyloxy-phenyl)-pyrrolo[2,3-d]pyrimidin-7-yl]-cyclobutylmethyl}-amide;
ethanesulfonic acid trans-{3-[4-amino-5-(3-benzyloxy-phenyl)-pyrrolo[2,3-d]pyrimidin-7-yl]-cyclobutylmethyl}-amide;
N-dimethyl-sulfamide trans-{3-[4-amino-5-(3-benzyloxy-phenyl)-pyrrolo[2,3-d]pyrimidin-7-yl]-cyclobutylmethyl}-amide;
N-dimethyl-sulfamide cis-{3-[4-amino-5-(3-benzyloxy-phenyl)-pyrrolo[2,3-d]pyrimidin-7-yl]-cyclobutylmethyl}-amide;
trans-{3-[4-amino-5-(3-benzyloxy-phenyl)-pyrrolo[2,3-d]pyrimidin-7-yl]-cyclobutylmethyl}-carbamic acid methyl ester;
cis-{3-[4-amino-5-(3-benzyloxy-phenyl)-pyrrolo[2,3-d]pyrimidin-7-yl]-cyclobutylmethyl}-carbamic acid methyl ester;
trans-{3-[4-amino-5-(3-benzyloxy-phenyl)-pyrrolo[2,3-d]pyrimidin-7-yl]-cyclobutylmethyl}-carbamic acid 2-methoxy-ethyl ester;
cis-{3-[4-amino-5-(3-benzyloxy-phenyl)-pyrrolo[2,3-d]pyrimidin-7-yl]-cyclobutylmethyl}-carbamic acid 2-methoxy-ethyl ester;
trans-1-{3-[4-amino-5-(3-benzyloxy-phenyl)-pyrrolo[2,3-d]pyrimidin-7-yl]-cyclobutylmethyl}-3-ethyl-urea;
cis-{3-[4-amino-5-(3-benzyloxy-phenyl)-pyrrolo[2,3-d]pyrimidin-7-yl]-cyclobutylmethyl}-3-ethyl-urea;
trans-1-{3-[4-amino-5-(3-benzyloxy-phenyl)-pyrrolo[2,3-d]pyrimidin-7-yl]-cyclobutylmethyl}-3-propyl-urea;
cis-1-{3-[4-amino-5-(3-benzyloxy-phenyl)-pyrrolo[2,3-d]pyrimidin-7-yl]-cyclobutylmethyl}-3-propyl-urea;
trans-1-{3-[4-amino-5-(3-benzyloxy-phenyl)-pyrrolo[2,3-d]pyrimidin-7-yl]-cyclobutylmethyl}-3-isopropyl-urea;
cis-1-{3-[4-amino-5-(3-benzyloxy-phenyl)-pyrrolo[2,3-d]pyrimidin-7-yl]-cyclobutylmethyl}-3-isopropyl-urea;
trans-1-{3-[4-amino-5-(3-benzyloxy-phenyl)-pyrrolo[2,3-d]pyrimidin-7-yl]-cyclobutylmethyl}-3-butyl-urea;
cis-1-{3-[4-amino-5-(3-benzyloxy-phenyl)-pyrrolo[2,3-d]pyrimidin-7-yl]-cyclobutylmethyl}-3-butyl-urea;
trans-1-{3-[4-amino-5-(3-benzyloxy-phenyl)-pyrrolo[2,3-d]pyrimidin-7-yl]-cyclobutylmethyl}-3-tert-butyl-urea;
cis-1-{3-[4-amino-5-(3-benzyloxy-phenyl)-pyrrolo[2,3-d]pyrimidin-7-yl]-cyclobutylmethyl}-3-tert-butyl-urea;
trans-1-{3-[4-amino-5-(3-benzyloxy-phenyl)-pyrrolo[2,3-d]pyrimidin-7-yl]-cyclobutylmethyl}-3-benzyl-urea;
trans-1-{3-[4-amino-5-(3-benzyloxy-phenyl)-pyrrolo[2,3-d]pyrimidin-7-yl]-cyclobutylmethyl}-3-(3-methyl-benzyl)-urea;
cis-1-{3-[4-amino-5-(3-benzyloxy-phenyl)-pyrrolo[2,3-d]pyrimidin-7-yl]-cyclobutylmethyl}-3-(3-methyl-benzyl)-urea;
cis-1-{3-[4-amino-5-(3-benzyloxy-phenyl)-pyrrolo[2,3-d]pyrimidin-7-yl]-cyclobutylmethyl}-3-(4-methoxy-benzyl)-urea;
trans-1-{3-[4-amino-5-(3-benzyloxy-phenyl)-pyrrolo[2,3-d]pyrimidin-7-yl]-cyclobutylmethyl}-3-(2-morpholin-4-yl-ethyl)-urea;
cis-1-{3-[4-amino-5-(3-benzyloxy-phenyl)-pyrrolo[2,3-d]pyrimidin-7-yl]-cyclobutylmethyl}-3-(2-morpholin-4-yl-ethyl)-urea;
trans-1-{3-[4-amino-5-(3-benzyloxy-phenyl)-pyrrolo[2,3-d]pyrimidin-7-yl]-cyclobutylmethyl}-3-(2-dimethylamino-ethyl)-urea;
cis-1-{3-[4-amino-5-(3-benzyloxy-phenyl)-pyrrolo[2,3-d]pyrimidin-7-yl]-cyclobutylmethyl}-3-(2-dimethylamino-ethyl)-urea;
trans-1-{3-[4-amino-5-(3-benzyloxy-phenyl)-pyrrolo[2,3-d]pyrimidin-7-yl]-cyclobutylmethyl}-3-(3-morpholin-4-yl-propyl)-urea;
cis-1-{3-[4-amino-5-(3-benzyloxy-phenyl)-pyrrolo[2,3-d]pyrimidin-7-yl]-cyclobutylmethyl}-3-(3-morpholin-4-yl-propyl)-urea;
trans-1-{3-[4-amino-5-(3-benzyloxy-phenyl)-pyrrolo[2,3-d]pyrimidin-7-yl]-cyclobutylmethyl}-3-(3-dimethylamino-propyl)-urea;
cis-1-{3-[4-amino-5-(3-benzyloxy-phenyl)-pyrrolo[2,3-d]pyrimidin-7-yl]-cyclobutylmethyl}-3-(3-dimethylamino-propyl)-urea;
trans-{3-[4-amino-5-(3-benzyloxy-phenyl)-pyrrolo[2,3-d]pyrimidin-7-yl]-cyclobutylmethyl}-urea;
cis-{3-[4-amino-5-(3-benzyloxy-phenyl)-pyrrolo[2,3-d]pyrimidin-7-yl]-cyclobutylmethyl}-urea;
trans-N-{3-[4-amino-5-(3-benzyloxy-phenyl)-pyrrolo[2,3-d]pyrimidin-7-yl]-cyclobutylmethyl}-acetamide;
cis-N-{3-[4-amino-5-(3-benzyloxy-phenyl)-pyrrolo[2,3-d]pyrimidin-7-yl]-cyclobutylmethyl}-acetamide;
trans-N-{3-[4-amino-5-(3-benzyloxy-phenyl)-pyrrolo[2,3-d]pyrimidin-7-yl]-cyclobutylmethyl}-isobutyramide;
cis-N-{3-[4-amino-5-(3-benzyloxy-phenyl)-pyrrolo[2,3-d]pyrimidin-7-yl]-cyclobutylmethyl}-isobutyramide;
trans-N-{3-[4-amino-5-(3-benzyloxy-phenyl)-pyrrolo[2,3-d]pyrimidin-7-yl]-cyclobutylmethyl}-2,2-dimethyl-propionamide;
cis-N-{3-[4-amino-5-(3-benzyloxy-phenyl)-pyrrolo[2,3-d]pyrimidin-7-yl]-cyclobutylmethyl}-2,2-dimethyl-propionamide;
trans-N-{3-[4-amino-5-(3-benzyloxy-phenyl)-pyrrolo[2,3-d]pyrimidin-7-yl]-cyclobutylmethyl}-2-piperidin-1-yl-acetamide;
cis-N-{3-[4-amino-5-(3-benzyloxy-phenyl)-pyrrolo[2,3-d]pyrimidin-7-yl]-cyclobutylmethyl}-2-piperidin-1-yl-acetamide;
trans-N-{3-[4-amino-5-(3-benzyloxy-phenyl)-pyrrolo[2,3-d]pyrimidin-7-yl]-cyclobutylmethyl}-2-morpholin-4-yl-acetamide;
cis-N-{3-[4-amino-5-(3-benzyloxy-phenyl)-pyrrolo[2,3-d]pyrimidin-7-yl]-cyclobutylmethyl}-2-morpholin-4-yl-acetamide;
trans-N-{3-[4-amino-5-(3-benzyloxy-phenyl)-pyrrolo[2,3-d]pyrimidin-7-yl]-cyclobutylmethyl}-2-(4-methyl-piperazin-1-yl)-acetamide;
cis-N-{3-[4-amino-5-(3-benzyloxy-phenyl)-pyrrolo[2,3-d]pyrimidin-7-yl]-cyclobutylmethyl}-2-(4-methyl-piperazin-1-yl)-acetamide;
trans-5-(3-benzyloxy-phenyl)-7-(3-morpholin-4-ylmethyl-cyclobutyl)-7H-pyrrolo[2,3-d]pyrimidin-4-ylamine;
trans-5-(3-benzyloxy-phenyl)-7-(3-piperidin-1-ylmethyl-cyclobutyl)-7H-pyrrolo[2,3-d]pyrimidin-4-ylamine;
trans-5-(3-benzyloxy-phenyl)-7-(3-pyrrolidin-1-ylmethyl-cyclobutyl)-7H-pyrrolo[2,3-d]pyrimidin-4-ylamine (ADW);
trans-5-(3-benzyloxy-phenyl)-7-[3-(4-methyl-piperazin-1-ylmethyl)-cyclobutyl]-7H-pyrrolo[2,3-d]pyrimidin-4-ylamine;
trans-7-[3-(adamantan-1-ylaminomethyl)-cyclobutyl]-5-(3-benzyloxy-phenyl)-7H-pyrrolo[2,3-d]pyrimidin-4-ylamine:
trans-1-{3-[4-amino-5-(3-benzyloxy-phenyl)-pyrrolo[2,3-d]pyrimidin-7-yl]-cyclobutylmethyl}-piperidin-4-ol;
trans-7-(3-azepan-1-ylmethyl-cyclobutyl)-5-(3-benzyloxy-phenyl)-7H-pyrrolo[2,3-d]pyrimidin-4-ylamine;
trans-5-(3-benzyloxy-phenyl)-7-[3-(2,5-dimethyl-pyrrolidin-1-ylmethyl)-cyclobutyl]-7H-pyrrolo[2,3-d]pyrimidin-4-ylamine;
trans-7-(3-azetidin-1-ylmethyl-cyclobutyl)-5-(3-benzyloxy-phenyl)-7H-pyrrolo[2,3-d]pyrimidin-4-ylamine;
trans-1-{3-[4-amino-5-(3-benzyloxy-phenyl)-pyrrolo[2,3-d]pyrimidin-7-yl]-cyclobutylmethyl}-piperidine-3-carboxylic acid amide;
trans-5-(3-benzyloxy-phenyl)-7-[3-(4-pyridin-2-yl-piperazin-1-ytmethyl)-cyclobutyl]-7H-pyrrolo[2,3-d]pyrimidin-4-ylamine;
trans-5-(3-benzyloxy-phenyl)-7-(3-thiomorpholin-4-ylmethyl-cyclobutyl)-7H-pyrrolo[2,3-d]pyrimidin-4-ylamine;
trans-5-(3-benryloxy-phenyl)-7-[3-(2,6-dimethyl-morpholin-4-ylmethyl)-cyclobutyl]-7H-pyrrolo[2,3-d]pyrimidin-4-ylamine;
trans-(S)-1-{3-[4-amino-5-(3-benzyloxy-phenyl)-pyrrolo[2,3-d]pyrimidin-7-yl]-cyclobutylmethyl}-pyrrolidine-2-carboxylic acid amide;
cis-7-(3-azepan-1-ylmethyl-cyclobutyl)-5-(3-benzyloxy-phenyl)-7H-pyrrolo[2,3-d]pyrimidin-4-ylamine;
cis-1-{3-[4-amino-5-(3-benzyloxy-phenyl)-pyrrolo[2,3-d]pyrimidin-7-yl]-cyclobutylmethyl}-piperidin-4-ol;
cis-4-{3-[4-amino-5-(3-benzyloxy-phenyl)-pyrrolo[2,3-d]pyrimidin-7-yl]-cyclobutylmethyl}-piperazine-1-carboxylic acid ethyl ester;
cis-5-(3-benzyloxy-phenyl)-7-[3-(4-phenyl-piperazin-1-ylmethyl)-cyclobutyl]-7H-pyrrolo[2,3-d]pyrimidin-4-ylamine;
cis-5-(3-benzyloxy-phenyl)-7-[3-(4-methyl-piperazin-1-ylmethyl)-cyclobutyl]-7H-pyrrolo[2,3-d]pyrimidin-4-ylamine;
cis-5-(3-benzyloxy-phenyl)-7-(3-thiomorpholin-4-ylmethyl-cyclobutyl)-7H-pyrrolo[2,3-d]pyrimidin-4-ylamine;
cis-5-(3-benzyloxy-phenyl)-7-[3-(2,6-dimethyl-morpholin-4-ylmethyl)-cyclobutyl]-7H-pyrrolo[2,3-d]pyrimidin-4-ylamine;
cis-(R)-1-{3-[4-amino-5-(3-benzyloxy-phenyl)-pyrrolo[2,3-d]pyrimidin-7-yl]-cyclobutylmethyl}-pyrrolidine-2-carboxylic acid amide;
cis-1-{3-[4-amino-5-(3-benzyloxy-phenyl)-pyrrolo[2,3-d]pyrimidin-7-yl]-cyclobutylmethyl}-piperidine-3-carboxylic acid amide;
trans-N-{3-[4-amino-5-(3-benzyloxy-phenyl)-pyrrolo[2,3-d]pyrimidin-7-yl]-cyclobutylmethyl}-2-ethoxy-acetamide;
trans-N-{3-[4-amino-5-(3-benzyloxy-phenyl)-pyrrolo[2,3-d]pyrimidin-7-yl]-cyclobutylmethyl}-2-(2-methoxy-ethoxy)-acetamide;
trans-1-{3-[4-amino-5-(3-benzyloxy-phenyl)-pyrrolo[2,3-d]pyrimidin-7-yl]-cyclobutylmethyl}-3-methyl-urea;
cis-1-{3-[4-amino-5-(3-benzyloxy-phenyl)-pyrrolo[2,3-d]pyrimidin-7-yl]-cyclobutylmethyl}-3-methyl-urea;
trans-pyrrolidine-1-carboxylic acid {3-[4-amino-5-(3-benzyloxy-phenyl)-pyrrolo[2,3-d]pyrimidin-7-yl]-cyclobutylmethyl}-amide;
trans-piperidine-1-carboxylic acid {3-[4-amino-5-(3-benzyloxy-phenyl)-pyrrolo[2,3-d]pyrimidin-7-yl]-cyclobutylmethyl}-amide;
trans-morpholine-4-carboxylic acid {3-[4-amino-5-(3-benzyloxy-phenyl)-pyrrolo[2,3-d]pyrimidin-7-yl]-cyclobutylmethyl}-amide;
trans-3-{3-[4-amino-5-(3-benzyloxy-phenyl)-pyrrolo[2,3-d]pyrimidin-7-yl]-cyclobutylmethyl}-1,1-dimethyl-urea;
trans-4-methyl-piperazine-1-carboxylic acid {3-[4-amino-5-(3-benzyloxy-phenyl)-pyrrolo[2,3-d]pyrimidin-7-yl]-cyclobutylmethyl}-amide;
trans-3-{3-[4-amino-5-(3-benzyloxy-phenyl)-pyrrolo[2,3-d]pyrimidin-7-yl]-cyclobutylmethyl}-1,1-diethyl-urea;
trans-{3-[4-amino-5-(3-benzyloxy-phenyl)-pyrrolo[2,3-d]pyrimidin-7-yl]-cyclobutylmethyl}-carbamic acid 2-diethylamino-ethyl ester;
trans-{3-[4-amino-5-(3-benzyloxy-phenyl)-pyrrolo[2,3-d]pyrimidin-7-yl]-cyclobutylmethyl}-carbamic acid 2-morpholin-4-yl-ethyl ester;
trans-{3-[4-amino-5-(3-benzyloxy-phenyl)-pyrrolo[2,3-d]pyrimidin-7-yl]-cyclobutylmethyl}-carbamic acid 2-(4-methyl-piperazin-1-yl)-ethyl ester;
trans-{3-[4-amino-5-(3-benzyloxy-phenyl)-pyrrolo[2,3-d]pyrimidin-7-yl]-cyclobutylmethyl}-carbamic acid 2-dimethylamino-ethyl ester;
trans-{3-[4-amino-5-(3-benzyloxy-phenyl)-pyrrolo[2,3-d]pyrimidin-7-yl]-cyclobutylmethyl}-carbamic acid ethyl ester;
trans-4-{3-[4-amino-5-(3-benzyloxy-phenyl)-pyrrolo[2,3-d]pyrimidin-7-yl]-cyclobutylmethyl}-piperazine-1-carboxylic acid ethyl ester;
cis-5-(3-benzyloxy-phenyl)-7-(3-pyrrolidin-1-ylmethyl-cyclobutyl)-7H-pyrrolo[2,3-d]pyrimidin-4-ylamine;
cis-7-(3-azetidin-1-ylmethyl-cyclobutyl)-5-(3-benzyloxy-phenyl)-7H-pyrrolo[2,3-d]pyrimidin-4-ylamine;
trans-3-[4-Amino-5-(3-benzyloxy-phenyl)-6-bromo-pyrrolo[2,3-d]pyrimidin-7-yl]-cyclobutanecarboxylic acid methylester;
trans-3-[4-Amino-5-(3-benzyloxy-phenyl)-6-methyl-pyrrolo[2,3-d]pyrimidin-T-yl]-cyclobutanecarboxylic acid methyl ester;
trans-{3-[4-Amino-5-(3-benzyloxy-phenyl)-6-methyl-pyrrolo[2,3-d]pyrimidin-7-yl]-cyclobutyl}-methanol;
cis-3-[4-Amino-5-(3-benzyloxy-phenyl)-6-bromo-pyrrolo[2,3-d]pyrimidin-7-yl]-cyclobutanecarboxylic acid methyl ester;
cis-3-[4-Amino-5-(3-benzyloxy-phenyl)-6-methyl-pyrrolo[2,3-d]pyrimidin-7-yl]-cyclobutanecarboxylic acid methyl ester;
cis-{3-[4-Amino-5-(3-benzyloxy-phenyl)-6-methyl-pyrrolo[2,3-d]pyrimidin-7-yl]-cyclobutyl}-methanol;
cis-3-[4-Amino-5-(3-benzyloxy-phenyl)-6-ethyl-pyrrolo[2,3-d]pyrimidin-7-yl]-cyclobutanecarboxylic acid methyl ester;
trans-3-[4-Amino-5-(3-benzyloxy-phenyl)-6-ethyl-pyrrolo[2,3-d]pyrimidin-7-yl]-cyclobutanecarboxylic acid methyl ester;
cis-{3-[4-Amino-5-(3-benzyloxy-phenyl)-6-ethyl-pyrrolo[2,3-d]pyrimidin-7-yl]-cyclobutyl}-methanol;
trans-{3-[4-Amino-5-(3-benzyloxy-phenyl)-6-ethyl-pyrrolo[2,3-d]pyrimidin-7-yl]-cyclobutyl}-methanol;
trans-5-(3-Benzyloxy-phenyl)-6-methyl-7-(3-pyrrolidin-1-ylmethyl-cyclobutyl)-7H-pyrrolo[2,3-d]pyrimidin-4-ylamine;
trans-5-(3-Benzyloxy-phenyl)-6-methyl-7-[3-(4-methyl-piperazin-1-ylmethyl)-cyclobutyl]-7H-pyrrolo[2,3-d]pyrimidin-4-ylamine;
trans-1-{3-[4-Amino-5-(3-benzyloxy-phenyl)-6-methyl-pyrrolo[2,3-d]pyrimidin-7-yl]-cyclobutylmethyl}-piperidin-4-ol;
trans-7-(3-Azetidin-1-ytmethyl-cyclobutyl)-5-(3-benzyloxy-phenyl)-6-methyl-7H-pyrrolo[2,3-d]pyrimidin-4-ylamine;
trans-5-(3-Benzyloxy-phenyl)-6-methyl-7-{3-[(tetrahydro-pyran-4-ylamino)-methyl]-cyclobutyl}-7H-pyrrolo[2,3-d]pyrimidin-4-ylamine;
trans-((R)-1-{3-[4-Amino-5-(3-benzyloxy-phenyl)-6-methyl-pyrrolo[2,3-d]pyrimidin-7-yl]-cyclobutylmethyl}-pyrrolidin-2-yl)-methanol;
cis-5-(3-Benzyloxy-phenyl)-6-methyl-7-(3-pyrrolidin-1-ylmethyl-cyclobutyl)-7H-pyrrolo[2,3-d]pyrimidin-4-ylamine;
cis-5-(3-Benzyloxy-phenyl)-6-methyl-7-[3-(4-methyl-piperazin-1-ylmethyl)-cyclobutyl]-7H-pyrrolo[2,3-d]pyrimidin-4-ylamine;
cis-7-(3-Azetidin-1-ylmethyl-cyclobutyl)-5-(3-benzyloxy-phenyl)-6-methyl-7H-pyrrolo[2,3-d]pyrimidin-4-ylamine;
cis-1-{3-[4-Amino-5-(3-benzyloxy-phenyl)-6-methyl-pyrrolo[2,3-d]pyrimidin-7-yl]-cyclobutylmethyl}-piperidin-4-ol;
cis-((R)-1-{3-[4-Amino-5-(3-benzyloxy-phenyl)-6-methyl-pyrrolo[2,3-d]pyrimidin-7-yl]-cyclobutylmethyl}-pyrrolidin-2-yl)-methanol;
cis-5-(3-Benzyloxy-phenyl)-6-ethyl-7-(3-pyrrolidin-1-ylmethyl-cyclobutyl)-7H-pyrrolo[2,3-d]pyrimidin-4-ylamine;
cis-5-(3-Benzyloxy-phenyl)-6-ethyl-7-[3-(4-methyl-piperazin-1-ylmethyl)-cyclobutyl]-7H-pyrrolo[2,3-d]pyrimidin-4-ylamine;
cis-7-(3-Azetidin-1-ylmethyl-cyclobutyl)-6-ethyl-5-{3-[(Z)-2-eth-(E)-ylidene-hexa-3,5-dienyloxy]-phenyl}-7H-pyrrolo[2,3-d]pyrimidin-4-ylamine;
cis-1-{3-[4-Amino-5-(3-benzyloxy-phenyl)-6-ethyl-pyrrolo[2,3-d]pyrimidin-7-yl]-cyclobutylmethyl}-piperidin-4-ol;
cis-((R)-1-{3-[4-Amino-5-(3-benzyloxy-phenyl)-6-ethyl-pyrrolo[2,3-d]pyrimidin-7-yl]-cyclobutylmethyl}-pyrrolidin-2-yl)-methanol; and
cis-5-(3-Benzyloxy-phenyl)-6-ethyl-7-{3-[(tetrahydro-pyran-4-ylamino)-methyl]-cyclobutyl}-7H-pyrrolo[2,3-d]pyrimidin-4-ylamine.

Among these compounds trans-5-(3-benzyloxy-phenyl)-7-(3-pyrrolidin-1-ylmethyl-cyclobutyl)-7H-pyrrolo[2,3-d]pyrimidin-4-ylamine (ADW) is most especially preferred. The preparation of these compounds is described in WO 02/092599.

Preferred PDGF receptor inhibitors to be used in accordance with the present invention are those described in EP 0 564 409 and most preferably 4-(4-methylpiperazin-1-ylmethyl)-N-[4-methyl-3-(4-pyridin-3-yl)-pyrimidin-2-ylamino)-phenyl]-benzamide (STI-571) or a salt thereof, especially the methane sulfonate salt in the β-crystal from as described in WO 99/03854.

The combinations of the present invention may be used for treating a range of diseases characterized by hyperproliferation. Such diseases include cancer for example, solid tumours, such as tumours from lung cancer, especially non-small cell lung cancer, prostate cancer, gastrointestinal stromal tumours and glioblastoma, as well as leukemias including chronic myeloid leukaemia (CML) and acute lymphoblastic leukaemia (ALL). Other proliferative diseases may be non-malignant such as atherosclerosis, thrombosis, psoriasis, sclerodermitis and fibrosis.

In addition, the synergistic effect of the combinations of the present invention such as exemplified here by STI-571 and ADW can show useful effects in the treatment of disorders arising as a result of transplantation, for example, allogenic transplantation, especially tissue rejection, such as especially obliterative bronchiolitis (OB), i.e. a chronic rejection of allogenic lung transplants. In contrast to patients without OB, those with OB often show an elevated PDGF concentration in bronchoalveolar lavage fluids. The combination of compounds can also be effective in treating diseases associated with vascular smooth-muscle cell migration and proliferation, such as restenosis and atherosclerosis. They may also be able of inhibiting angiogenesis. All these hyperproliferative conditions are described in detail in EP 0 564 409, WO 97/02266, WO 99/03854 and WO 98/35958, or the other above-mentioned references.

While not wishing to be limited by theory, one aspect of the synergistic activity observed when tissue specific and tissue non-specific growth factor receptor inhibitors are combined may include inhibiting cell growth to a particular phase in the cell cycle with a first inhibitor and then starving those growth inhibited cells with a second inhibitor. STI-571 is a recognized growth inhibitor that halts the growth of tumour cells. In some cases, the tumour can adapt and bypass the growth inhibition. By addition of a cytotoxic agent with specificity for growth arrested cells, the ability of the tumour to bypass the growth inhibition is impeded. We propose a role for tissue non-specific growth factor receptor activation inhibitors as cytotoxic agents for targeting cells in which the cell replication cycle has been halted.

For example, STI-571 is one example of an inhibitor of tissue specific growth factor receptor activation that is a potent inhibitor of bcr/abl, an oncogenic fusion protein that causes CML. However, it has been observed in an ongoing clinical trial that CML patients in blast crisis and relapsed Philadelphia Chromosome Positive Acute Lymphoblastic Leukemia (Ph+-ALL) patients show only temporary responses to STI-571, which are followed in a brief period of time by the development of resistance. It is expected that resistance may be avoided when STI-571 is co-administered with a tissue non-specific growth factor receptor inhibitor such as the IGF1 receptor inhibitor ADW.

The synergistic inhibition of cell growth by combined inhibition of platelet-derived growth factor receptor signalling and insulin-like growth factor signalling was demonstrated *in vitro* by observing the effect of small molecule signal transduction inhibitors STI-571 and ADW on sis 3T3 cells, ras 3T3 cells and U343 glioma cells.

Simian sarcoma virus-transformed NIH/3T3 (sis 3T3) fibroblast cells secrete a potent growth-promoting activity identical with the platelet-derived growth factor in mitogenic assays. The secreted p28v-sis appears to stimulate autocrine cell growth of sis-transformed cells via activation of the PDGF receptor.

In contrast to the sis 3T3 cells in which a secreted protein acts through a kinase at the cell receptor, we have shown that ADW and STI-571 either singly or together have little effect on cell growth of ras 3T3 cells. This is not surprising because although the ras proteins are necessary and sufficient for insulin-induced activation of cytosolic kinases (Raf-1, MAPK, and RSK), they do not interact with, and are not inhibited by, either of these drugs. The major determinant of membrane-bound Ras activity is its ability to bind guanine nucleotides GDP and GTP. http://ethesis.helsinki.fi/julkaisut/laa/haart/vk/kivinen/review,html#1

In a first aspect, the present invention relates to a combination, such as a combined preparation or a pharmaceutical composition, which comprises (a) an inhibitor of a tissue non-specific growth factor receptor, especially those mentioned hereinbefore, in particular those mentioned as being preferred, and (b) an inhibitor of a tissue specific growth factor receptor, especially those mentioned hereinbefore, in particular those mentioned as being preferred, in which the active ingredients (a) and (b) are present in each case in free form or in the form of a pharmaceutically acceptable salt, for simultaneous, concurrent, separate or sequential use.

The term "a combined preparation" defines especially a "kit of parts" in the sense that the combination partners (a) and (b) as defined above can be dosed independently or by use of different fixed combinations with distinguished amounts of the combination partners (a) and (b), i.e., simultaneously, concurrently, separately or sequentially. The parts of the kit of parts can then, e.g., be administered simultaneously or chronologically staggered, that is at different time points and with equal or different time intervals for any part of the kit of parts.

The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, severity of the disease, age, sex, body weight, etc. of the patients.

In a preferred aspect, the present invention relates to a combination which comprises (a) an inhibitor of a tissue non-specific growth factor receptor, especially those mentioned hereinbefore, in particular those mentioned as being preferred, or a pharmaceutically acceptable salt thereof, and (b) an inhibitor of a tissue specific growth factor receptor, especially those mentioned hereinbefore, in particular those mentioned as being preferred, or a pharmaceutically acceptable salt thereof, for use in the treatment of glioblastoma.

In the context of the present invention the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition.

Within the context of this disclosure, any reference to an inhibitor of a tissue non-specific growth factor receptor or to an inhibitor of a tissue specific growth factor receptor is understood to include said compounds in their free form or as salts, especially pharmaceutically acceptable salts, or any crystal forms thereof including hydrates or solvates, if not indicated otherwise and where appropriate and expedient.

In another aspect, the present invention relates to the use of an inhibitor of a tissue non-specific growth factor receptor, especially those mentioned hereinbefore, in particular those mentioned as being preferred, or a pharmaceutically acceptable salt thereof, for the preparation of a medicament, for use in combination with an inhibitor of a tissue specific growth factor receptor, especially those mentioned hereinbefore, in particular those mentioned as being preferred, or a pharmaceutically acceptable salt thereof, for the treatment of glioblastoma.

The present invention also relates to the use of an inhibitor of a tissue specific growth factor receptor, especially those mentioned hereinbefore, in particular those mentioned as being preferred, or a pharmaceutically acceptable salt thereof, for the preparation of a medicament, for use in combination with an inhibitor of a tissue non-specific growth factor receptor, especially those mentioned hereinbefore, in particular those mentioned as being preferred, or a pharmaceutically acceptable salt thereof, for the treatment of glioblastoma.

In a further aspect, the present invention relates to pharmaceutical compositions comprising (a) one or more unit dosage forms of an inhibitor of a tissue non-specific growth factor receptor, especially those mentioned hereinbefore, in particular those mentioned as being preferred, or a pharmaceutically acceptable salt thereof, and (b) one or more unit dosage forms of an inhibitor of a tissue specific growth factor receptor, especially those mentioned hereinbefore, in particular those mentioned as being preferred, or a pharmaceutically acceptable salt thereof, together with at least one pharmaceutically acceptable carrier.

The invention also relates to the use of a combination of the present invention for the preparation of a pharmaceutical composition for the treatment of glioblastoma.

The present invention further relates to a commercial package or product comprising (a) an inhibitor of a tissue non-specific growth factor receptor, especially those mentioned hereinbefore, in particular those mentioned as being preferred, or a pharmaceutically acceptable salt thereof, and (b) an inhibitor of a tissue specific growth factor receptor. especially those mentioned hereinbefore, in particular those mentioned as being preferred, or a pharmaceutically acceptable salt thereof, together with instructions for simultaneous, concurrent, separate or sequential use thereof in the treatment of glioblastoma.

The present invention also relates to a commercial package or product comprising an inhibitor of a tissue non-specific growth factor receptor, especially those mentioned hereinbefore, in particular those mentioned as being preferred, or a pharmaceutically acceptable salt thereof, together with instructions for use in combination with an inhibitor of a tissue specific growth factor receptor, especially those mentioned hereinbefore, in particular those mentioned as being preferred, or a pharmaceutically acceptable salt thereof, for the treatment of glioblastoma,
or
a commercial package or product comprising an inhibitor of a tissue specific growth factor receptor, especially those mentioned hereinbefore, in particular those mentioned as being preferred, or a pharmaceutically acceptable salt thereof, together with Instructions for use in combination with an inhibitor of a tissue non-specific growth factor receptor, especially those mentioned hereinbefore, in particular those mentioned as being preferred, or a pharmaceutically acceptable salt thereof, for the treatment of glioblastoma.

According to the present invention, a patient is treated with therapeutically effective amounts of an inhibitor of a tissue non-specific growth factor receptor and an inhibitor of a tissue specific growth factor receptor in order to treat glioblastoma, each according to a dosage regimen that is appropriate for the individual agent. One of skill in the art has the ability to determine appropriate pharmaceutically effective amounts of the combination components.

The inhibitors of the tissue specific and non-specific growth factor receptors can be prepared and administered as described in the art such as in the documents cited above. If they are available on the market they can be administered for example in the form as marketed.

The administration of a pharmaceutical formulation of the present invention can be achieved as follows:

*Dosage*: The pharmaceutical compositions comprise from approximately 0.0001 % to approximately 95 % of the active components in the formulation, dosage forms that are in single dose form preferably include from approximately 10 % to approximately 90 % of the formulation, and dosage forms that are not in single dose form preferably comprising from approximately 10 % to approximately 60 % of each component. Unit dose forms, such as dragees, tablets, ampoules or capsules, comprise from approximately 5 mg to approximately 1.5 g of active agents preferably from 5 mg to approximately 1 g.

The dose of the active agent depends on the species of the warm-blooded animal to be treated, its body weight, its age and individual status, individual pharmacokinetic circumstances, the disease to be treated and the administration route. Preferably, for a body weight of approximately 70 kg a daily dose of from 10 mg to 2500 mg, more preferably from approximately 50 mg to approximately 2000 mg, most preferably from approximately 100 mg to approximately 1500 mg, of any one of component (a) and/or (b) is administered. Children receive a correspondingly lower dose based on their skin surface area (the skin surface area of an adult of 70 kg as reference is 1.73 m²).

*Route of delivery*: The formulation may be administered orally, transdermally including by submucosal administration or parenterally.

*Formulation*: The pharmaceutical compositions are prepared in a manner known per se, for example by means of conventional mixing, granulating, confectioning, dissolving or lyophilising processes. For example pharmaceutical compositions for oral administration can be obtained by combining the active components with one or more solid or liquid carriers, where necessary granulating a resulting mixture and processing the mixture or the granules, if desired or appropriate with the addition of further excipients, to form tablets or dragee cores or solutions, respectively.

Suitable carriers are especially fillers, such as sugars, e. g. lactose, saccharose, mannitol or sorbitol, cellulose preparations and/or calcium phosphates, e. g. tricalcium phosphate or calcium hydrogen phosphate, and binders, such as starches, e. g. corn, wheat, rice or potato starch, methylcellulose, hydroxypropylmethylcellulose, sodium carboxymethylcellulose and/or polyvinylpyrrolidone, and/or, if desired, disintegrators, such as the above-mentioned starches, and also carboxymethyl starch, crosslinked polyvinylpyrrolidone or alginic acid or a salt thereof, such as sodium alginate. Additional excipients are especially flow conditioners and lubricants, e. g. silicic acid, talc, stearic acid or salts thereof, such as magnesium or calcium stearate, and/or polyethylene glycol, or derivatives thereof.

Dragee cores may be provided with suitable, optionally enteric, coatings, there being used, *inter alia,* concentrated sugar solutions which may comprise gum arabic, talc, polyvinylpyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropylmethylcellulose phthalate. Dyes or pigments may be added to the tablets or dragee coatings, e. g. for identification purposes or to indicate different doses of active ingredient.

Orally administrable pharmaceutical compositions are also dry-filled capsules consisting of gelatin, and also soft sealed capsules consisting of gelatin and a plasticiser, such as glycerol or sorbitol. The dry-filled capsules may contain component (a) and/or (b) in the form of granules, for example in admixture with fillers, such as corn starch, binders and/or glidants, such as talcum or magnesium stearate, and, where appropriate, stabilisers (see above for "suitable carriers"). In soft capsules, the active ingredient is preferably dissolved or suspended in suitable liquid excipients, e. g. fatty oils, 'Lauroglycol (Gattefoss6 S. A., Saint Priest, France), egelucire (Gattefoss S. A., Saint Priest, France) or sesame oil, paraffin oil or liquid polyethylene glycols, such as PEG 300 or 400 (Fluka, Switzerland), or polypropylene glycols, to each of which stabilisers or detergents may also be added, or in water comprising further soluble carriers as mentioned above, such as methylcellulose or mannitol.

Other oral forms of administration are, for example, solutions or syrups prepared in customary manner that comprise the active compositions e. g. in suspended form and in a concentration of approximately from 0.001 % to 20 %, preferably approximately 0.001% to about 2%, or in a similar concentration that provides a suitable single dose when administered, for example, in measures of 0.5 to 10 ml. Also suitable, for example, are powdered or liquid concentrates for preparing shakes, e.g. in milk. Such concentrates can also be packed in single-dose quantities.

Transdermal Delivery Systems are possible, especially with neutral active ingredients. Suitable formulations include, for example, about 0.0001 % to about 2% by weight of the active ingedients. For example, there are provided formulations which include about 2 % to 99.9999 % (or the balance to 100 %) of a short chain aliphatic alcohol. Suitable alcohols include ethanol, isopropanol, propylene glycol and glycerol. These formulations may additionally include a flux enhancer. Suitable flux enhancers include, for example, decylmethylsulfoxide, dimethylsufoxide as well as cyclic ketones, lactones, anhydrides and esters. Some of these flux enhancers also increase retention of the active agents and thus act to increase the concentration of it in the skin itself. For formulations for direct (local) treatment, such as topical application to the skin, it is preferred to use a flux enhancer, which not only maximizes transdermal flux, but increases retention of component (a) and/or (b) in the skin. Certain cyclic ketone and lactone enhancers have been reported to increase local retention as well and, thus, comprise a preferred class of enhancers for topical administration of the active agents. In formulations for systemic treatment, it is preferable to use a flux enhancer, which maximizes flux with a minimal local retention of the active ingredient.

Suitable rectally administrable pharmaceutical compositions are e. g. suppositories that consist of a combination of the active ingredient with a suppository base. Suitable suppository bases are e. g. natural or synthetic triglycerides, paraffin hydrocarbons, polyethylene glycols or higher alkanols.

For parenteral administration there are suitable, especially, aqueous solutions of an active ingredient in water-soluble form, e. g. in the form of a water-soluble salt, in the presence or absence of salts, such as sodium chloride, and/or sugar alcohols, such as mannitol, or aqueous injection suspensions that comprise viscosity-increasing substances, e. g. sodium carboxymethylcellulose, sorbitol and/or dextran, and, where appropriate, stabilisers. The active agents where appropriate together with excipients, may also be in the form of a lyophilisate and may be made into a solution prior to parenteral administration by the addition of suitable solvents. Solutions as used e. g. for parenteral administration may also be used as infusion solutions.

Formulations include any active agent that are customary for the respective clinical use of any one or more agents belonging to that group of compounds which are known in the art. Pathological conditions described herein may be treated with a combination of active agents, or their pharmaceutical acceptable salts. The formulations may be administered prophylactically or therapeutical, in an effective amount for a warm-blooded animal, e. g. man, requiring such treatment, preferably in the form of a pharmaceutical composition.

### Examples: Synergistic effect on cell growth of combined inhibition of platelet-derived growth factor receptor signalling and insulin-like growth factor signalling using the small molecule signal transduction inhibitors STI-571 and ADW

*Screening Assay*: An assay for efficacy of various dose combinations of STI-571 and ADW was performed using 12-well tissue culture dishes with 5 x 10³ cells plated/well. Cells were plated and attached overnight in growth media consisting of DMEM with 10% fetal bovine serum (FBS). Media was replaced with various dose combinations of STI-571 (0 µM, 1 µM, 3 µM), and ADW (0 µM, 0.1 µM, 0.3 µM, 1 µM) in DMEM + 5% platelet-poor plasma (PPP). Media with agents was replaced every 48 hours. At day 5 and 8, media was withdrawn and cells were fixed and stained in 0.5% crystal violet in 70% methanol. The results are shown in Figure 1. The crystal violet stained cells reveal a dense intact monolayer in media absent either agent. In the presence of increasing amounts of STI-571, the density of cells in the monolayer is visibly depleted. A similar effect is observed for ADW on its own. However, cell growth depletion is dramatically enhanced at intermediate levels of STI-571 (1.0 µM) when even small amounts of ADW is additionally supplied to the cells. Almost no cells are observed at 1 µM STI-571 and 0.3 µM ADW. This observed synergistic effect is an important finding in the treatment of diseases characterized by hyperproliferation of cells.

*Quantitative Growth Inhibition Assay*: For quantitative assessment of cell growth inhibition, the effect of combined treatment with STI-571 and ADW was measured either through direct counting of remaining cell number (Figure 2), or with a cell viability assay based on reduction of an MTS tetrazolium compound resulting in a water-soluble formazan absorbing light at 490 nM (Promega CellTiter Aqueous One Solution Cell Proliferation Assay) (Figure 3 and 4). For cell-counting assays, 5 x 10³ cells were plated in triplicate in 100 mm cell culture dishes and allowed to attach overnight as above in DMEM + 10% FBS and treated with agents in DMEM + 5%PPP. Media and agents were replaced every 48 hours. At day 5 cells were trypsinized and counted with hemacytometer. Figure 2 shows the dramatically decreased number of surviving cells when ADW is used in conjunction with STI-571 in U343 human glioma cells (Fig. 2).

For the MTS-based cell-viability assay, 5 x 10³ cells were plated in 24 well dishes overnight in DMEM + 10% FBS. Media was then replaced with DMEM + 5% PPP containing appropriate concentrations of STI-571 and/or ADW. Each condition was assayed in triplicate. Media and agents were replaced every 48 hours and viable surviving fraction estimated using the MTS assay at day 5 following treatment. Figure 3 shows that the combined treatment with ADW and STI-571 in sis 3T3 fibroblasts results in increased cell-killing (Fig. 3A). Control ras-transformed fibroblasts (ras 3T3 cells) show decreased survival following treatment with ADW but show no effect on survival with STI-571 administered alone or in combination with ADW over the dose range examined (Fig. 3B).

In Figure 4, the concentration of STI-571 varies and the concentration of ADW is constant at 1 µM (lower line) or zero µM (upper line). A dramatic synergistic effect is observed.

## Claims

1. Use of an inhibitor of insulin-like growth factor 1 receptor, or a pharmaceutically acceptable salt thereof, for the preparation of a medicament for simultaneous, concurrent, separate or sequential use in combination with an inhibitor of platelet-derived growth factor receptor, or a pharmaceutically acceptable salt thereof, for the treatment of glioblastoma.

2. Use of an inhibitor of platelet-derived growth factor receptor, or a pharmaceutically acceptable salt thereof, for the preparation of a medicament for simultaneous, concurrent, separate or sequential use in combination with an inhibitor of insulin-like growth factor 1 receptor, or a pharmaceutically acceptable salt thereof, for the treatment of glioblastoma.

3. The use according to claim 1 or 2 wherein the insulin-like growth factor 1 receptor inhibitor is trans-5-(3-benzyloxy-phenyl)-7-(3-pyrrolidin-1-ylmethyl-cyclobutyl)-7H-pyrrolo[2,3-d]pyrimidin-4-ylamine or a salt thereof.

4. The use according to claim 1 or 2 wherein the insulin-like growth factor 1 receptor inhibitor is tyrphostin or a salt thereof.

5. The use according to any one of claims 1 to 4 wherein the platelet-derived growth factor receptor inhibitor is 4-(4-methylpiperazin-1-ylmethyl)-N-[4-methyl-3-(4-pyridin-3-yl)-pyrimidin-2-ylamino)-phenyl]-benzamide or a salt thereof.

6. The use according to claim 1 or 2 wherein the inhibitor of insulin-like growth factor 1 receptor is trans-5-(3-benzyloxy-phenyl)-7-(3-pyrrolidin-1-ylmethyl-cyclobutyl)-7H-pyrrolo[2,3-d]pyrimidin-4-ylamine, or a pharmaceutically acceptable salt thereof, and the inhibitor of platelet-derived growth factor receptor is 4-(4-methylpiperazin-1-ylmethyl)-N-[4-methyl-3-(4-pyridin-3-yl)-pyrimidin-2-ylamino)-phenyl]-benzamide, or a pharmaceutically acceptable salt thereof.

7. A pharmaceutical composition for the treatment of glioblastoma which comprises (a) one or more unit dosage forms of an inhibitor of insulin-like growth factor 1 receptor, or a pharmaceutically acceptable salt thereof, and (b) one or more unit dosage forms of an inhibitor of platelet-derived growth factor receptor, or a pharmaceutically acceptable salt thereof, together with at least one pharmaceutically acceptable carrier.

8. The pharmaceutical composition of claim 7 which comprises (a) one or more unit dosage forms of an inhibitor of insulin-like growth factor 1 receptor of claim 3 or 4, or a pharmaceutically acceptable salt thereof, and (b) one or more unit dosage forms of the inhibitor of platelet-derived growth factor receptor of claim 5, or a pharmaceutically acceptable salt thereof.

9. A commercial package or product comprising (a) an inhibitor of insulin-like growth factor 1 receptor, or a pharmaceutically acceptable salt thereof, and (b) an inhibitor of platelet-derived growth factor receptor, or a pharmaceutically acceptable salt thereof, together with instructions for simultaneous, concurrent, separate or sequential use thereof in the treatment of glioblastoma.

10. The commercial package or product of claim 9 comprising (a) an inhibitor of insulin-like growth factor 1 receptor of claim 3 or 4, or a pharmaceutically acceptable salt thereof, and (b) the inhibitor of platelet-derived growth factor receptor of claim 5, or a pharmaceutically acceptable salt thereof.

## Patentansprüche

1. Verwendung eines Inhibitors für den insulinähnlichen Wachstumsfaktor 1-Rezeptor oder eines pharmazeutisch akzeptablen Salzes hiervon, zur Herstellung eines Arzneimittels für eine simultane, gemeinsame, separate oder sequentielle Verwendung in Kombination mit einem Inhibitor für einen von Blutplättchen abgeleiteten Wachstumsfaktor-Rezeptor oder eines pharmazeutisch akzeptablen Salzes hiervon für die Behandlung eines Glioblastoms.

2. Verwendung eines Inhibitors für einen von Blutplättchen abgeleiteten Wachstumsfaktor-Rezeptor oder eines pharmazeutisch akzeptablen Salzes hiervon, zur Herstellung eines Arzneimittels für die simultane, gemeinsame, separate oder sequentielle Verwendung in Kombination mit einem Inhibitor für einen insulinähnlichen Wachstumsfaktor-1-Rezeptor oder eines pharmazeutisch akzeptablen Salzes hiervon für die Behandlung eines Glioblastoms.

3. Verwendung nach Anspruch 1 oder 2, worin der Inhibitor für den insulinähnlichen Wachstumsfaktor-1-Rezeptor trans-5-(3-Benzyloxyphenyl)-7-(3-pyrrolidin-1-ylmethylcyclobutyl)-7H-pyrrolo[2,3-d]pyrimidin-4-ylamin oder ein Salz hiervon ist.

4. Verwendung nach Anspruch 1 oder 2, worin der Inhibitor für den insulinähnlichen Wachstumsfaktor-1-Rezeptor Tyrphostin oder ein Salz hiervon ist.

5. Verwendung nach einem der Ansprüche 1 bis 4, worin der Inhibitor für den von Bluttplättchen abgeleiteten Wachstumsfaktor-Rezeptor 4-(4-Methylpiperazin-1-ylmethyl)-N-[4-methyl-3-(4-pyridin-3-yl)-pyrimidin-2-ylamino)-phenyl]benzamid oder ein Salz hiervon ist.

6. Verwendung nach Anspruch 1 oder 2, worin der Inhibitor für den insulinähnlichen Wachstumsfaktor-1-Rezeptor trans-5-(3-Benzyloxyphenyl)-7-(3-pyrrolidin-1-ylmethylcyclobutyl)-7H-pyrrolo[2,3-d]pyrimidin-4-ylamin oder ein pharmazeutisch akzeptables Salz hiervon ist und der Inhibitor für den von Bluttplättchen abgeleiteten Wachstumsfaktor-Rezeptor 4-(4-Methylpiperazin-1-ylmethyl)-N-[4-methyl-3-(4-pyridin-3-yl)-pyrimidin-2-ylamino)phenyl]benzamid oder ein pharmazeutisch akzeptables Salz hiervon ist.

7. Pharmazeutische Zusammensetzung zur Behandlung eines Glioblastoms, umfassend (a) eine oder mehrere Einheitsdosierungsformen eines Inhibitors für den insulinähnlichen Wachstumsfaktor-1-Rezeptor oder eines pharmazeutisch akzeptablen Salzes hiervon und (b) eine oder mehrere Einheitsdosierungsformen eines Inhibitors für den von Bluttplättchen abgeleiteten Wachstumsfaktor-Rezeptor oder ein pharmazeutisch akzeptables Salz hiervon, zusammen mit wenigstens einem pharmazeutisch akzeptablen Träger.

8. Pharmazeutische Zusammensetzung nach Anspruch 7, umfassend (a) eine oder mehrere Einheitsdosierungsformen eines Inhibitors für den insulinähnlichen Wachstumsfaktor-1-Rezeptor nach Anspruch 3 oder 4 oder eines pharmazeutisch annehmbaren Salzes hiervon und (b) eine oder mehrere Einheitsdosierungsformen des Inhibitors für den von Bluttplättchen abgeleiteten Wachstumsfaktor-Rezeptors von Anspruch 5 oder eines pharmazeutisch akzeptablen Salzes hiervon.

9. Handelspackung oder Produkt, umfassend (a) einen Inhibitor für den insulinähnlichen Wachstumsfaktor-1-Rezeptor oder einem pharmazeutisch akzeptablen Salz hiervon und (b) einen Inhibitor für einen von Bluttplättchen abgeleiteten Wachstumfaktor-Rezeptor oder einem pharmazeutisch akzeptablen Salz hiervon, zusammen mit Instruktionen zur gleichzeitigen, gemeinsamen, separaten oder sequentiellen Anwendung hiervon zur Behandlung eines Glioblastoms.

10. Handelspackung oder Produkt nach Anspruch 9, umfassend (a) einen Inhibitor für den insulinähnlichen Wachstumsfaktor-1-Rezeptor nach Anspruch 3 oder 4 oder ein pharmazeutisch akzeptables Salz hiervon und (b) den Inhibitor für den von Bluttplättchen abgeleiteten Wachstumsfaktor-Rezeptor nach Anspruch 5, oder ein pharmazeutisch akzeptables Salz hiervon.

## Revendications

1. Utilisation d'un inhibiteur du récepteur du facteur de croissance 1 analogue à l'insuline, ou de l'un de ses sels pharmaceutiquement acceptables, pour la préparation d'un médicament pour une utilisation simultanée, concurrente, distincte ou séquentielle en combinaison avec un inhibiteur du récepteur du facteur de croissance dérivé des plaquettes, ou l'un de ses sels pharmaceutiquement acceptables, pour le traitement du glioblastome.

2. Utilisation d'un inhibiteur du récepteur du facteur de croissance dérivé des plaquettes, ou de l'un de ses sels pharmaceutiquement acceptables, pour la préparation d'un médicament pour une utilisation simultanée, concurrente, distincte ou séquentielle en combinaison avec un inhibiteur du récepteur du facteur de croissance 1 analogue à l'insuline, ou l'un de ses sels pharmaceutiquement acceptables, pour le traitement du glioblastome.

3. Utilisation selon la revendication 1 ou 2, dans laquelle l'inhibiteur du facteur de croissance 1 de type insuline est la trans-5-(3-benzyloxy-phényl)-7-(3-pyrrolidin-1-ylméthyl-cyclobutyl)-7H-pyrrolo[2,3-d]pyrimidin-4-ylamine, ou l'un de ses sels.

4. Utilisation selon la revendication 1 ou 2, dans laquelle l'inhibiteur du récepteur du facteur de croissance 1 analogue à l'insuline est la tyrphostine ou l'un de ses sels.

5. Utilisation selon l'une quelconque des revendications 1 à 4, dans laquelle l'inhibiteur du récepteur du facteur de croissance dérivé des plaquettes est le 4-(4-méthylpipérazin-1-ylméthyl)-N-[4-méthyl-3-(4-pyridin-3-yl)-pyrimidin-2-ylamino)-phényl]-benzamide ou l'un de ses sels.

6. Utilisation selon la revendication 1 ou 2, dans laquelle l'inhibiteur du récepteur du facteur de croissance 1 analogue à l'insuline est la trans-5-(3-benzyloxy-phényl)-7-(3-pyrrolidin-1-ylméthyl-cyclobutyl)-7H-pyrrolo[2,3-d]pyrimidin-4-ylamine, ou l'un de ses sels pharmaceutiquement acceptables, et l'inhibiteur du récepteur du facteur de croissance dérivé des plaquettes est le 4-(4-méthylpipérazin-1-ylméthyl)-N-[4-méthyl-3-(4-pyridin-3-yl)-pyrimidin-2-ylamino)-phényl]-benzamide, ou l'un de ses sels pharmaceutiquement acceptables.

7. Composition pharmaceutique pour le traitement du glioblastome, qui comprend (a) une ou plusieurs formes posologiques unitaires d'un inhibiteur du récepteur du facteur de croissance 1 analogue à l'insuline, ou l'un de ses sels pharmaceutiquement acceptables, et (b) une ou plusieurs formes posologiques unitaires d'un inhibiteur du récepteur du facteur de croissance dérivé des plaquettes, ou l'un de ses sels pharmaceutiquement acceptables, conjointement avec au moins un support pharmaceutiquement acceptable.

8. Composition pharmaceutique selon la revendication 7, qui comprend (a) une ou plusieurs formes posologiques unitaires d'un inhibiteur du récepteur du facteur de croissance 1 analogue à l'insuline selon la revendication 3 ou 4, ou l'un de ses sels pharmaceutiquement acceptables, et (b) une ou plusieurs formes posologiques de l'inhibiteur du récepteur du facteur de croissance dérivé des plaquettes selon la revendication 5, ou l'un de ses sels pharmaceutiquement acceptables.

9. Conditionnement ou produit commercial comprenant (a) un inhibiteur du récepteur du facteur de croissance 1 analogue à l'insuline, ou l'un de ses sels pharmaceutiquement acceptables, et (b) un inhibiteur du récepteur du facteur de croissance dérivé des plaquettes, ou l'un de ses sels pharmaceutiquement acceptables, conjointement avec un mode d'emploi pour une utilisation simultanée, concurrente, distincte ou séquentielle de celui-ci dans le traitement du glioblastome.

10. Conditionnement ou produit commercial selon la revendication 9, comprenant (a) l'inhibiteur du récepteur du facteur de croissance 1 analogue l'insuline selon la revendication 3 ou 4, ou l'un de ses sels pharmaceutiquement acceptables, et (b) l'inhibiteur du récepteur du facteur de croissance dérivé des plaquettes selon la revendication 5, ou l'un de ses sels pharmaceutiquement acceptables.
